# EUROPEAN PATENT APPLICATION

(11) **EP 1 777 219 A1**
(43) Date of publication of application: **25.04.2007**
(21) Application number: 05292212.7
(22) Date of filing: 20.10.2005
(51) Int. Cl.: C07D 295/08, C07D 295/12, C07C 211/57, A61K 31/4453, A61K 31/495, A61K 31/5375

(54) **1,2-Substituted naphthalene derivatives with antiglutamatergic properties and uses thereof**

(71) Applicant: Faust Pharmaceuticals, 67400 Illkirch (FR)
(72) Inventor: Schann, Stephan, 67118 Geispolsheim (FR); Mayer, Stanislas, 67114 Eschau (FR); Foucher, Laurence, 67400 Illkirch (FR)

(57) **Abstract**

The present invention provides new compounds displaying antiglutamatergic properties. It also provides the use of such compounds for the treatment and/or prophylaxis of conditions and diseases linked to abnormalities in glutamatergic transmission. It further provides a pharmaceutical composition for the treatment and/or prophylaxis of acute and chronic neurodegenerative conditions and disorders.

## Description

The present invention provides new compounds displaying antiglutamatergic properties. It also provides the use of such compounds for the treatment and/or prophylaxis of conditions and diseases linked to abnormalities in glutamatergic transmission. It further provides a pharmaceutical composition for the treatment and/or prophylaxis of acute and chronic neurodegenerative conditions and disorders.

In the central nervous system (CNS), glutamate is the main excitatory neurotransmitter and is referred to as an excitatory amino-acid, and gamma-aminobutyric acid (GABA) is the main inhibitory neurotransmitter. The balance between excitation and inhibition is of utmost importance to CNS functions, and dysfunctions of either of the two can be related to various neurological disorders.

Glutamate binds or interacts with one or more glutamate receptors, which can be differentiated pharmacologically into different classes and subtypes. Glutamate is ubiquitously distributed in the nervous system in high concentrations, especially in the brain and spinal cord of mammals, where it is working at a variety of excitatory synapses being thereby involved in virtually all physiological functions such as motor control, vision, central control of heart, processes of learning and memory. However, a large number of studies have established that cellular communication involving glutamate can also lead to a mechanism of neuronal cell damage or loss. This mechanism is known as excitotoxicity. Excitotoxicity has a role in acute disorders (traumatic, anoxic, hypoglycaemic or vascular disorders), as well as in chronic neurodegenerative disorders.

Glutamatergic transmission has been shown to be clearly involved in the pathophysiology of a number of neuronal damages and injuries. Many nervous system disorders including epilepsy and chronic or acute degenerative processes such as for example Alzheimer's disease, Huntington's disease, Parkinson's disease and amyotrophic lateral sclerosis (Mattson et al., Neuromolecular Med., 65, 2003), but also AIDS-induced dementia, multiple sclerosis, spinal muscular atrophy, retinopathy, stroke, ischemia, hypoxia, hypoglycaemia and various traumatic brain injuries, involve neuronal cell death caused by imbalanced levels of glutamate. It has also been shown that drug-induced neurotoxicity, for example neurotoxic effects of methamphetamine (METH) on striatal dopaminergic neurons, could actually be mediated by over-stimulation of the glutamate receptors (Stephans and Yamamoto, 1994, Synapse, 17, 203-209). Antidepressant and anxiolytic-like effects of compounds acting on glutamate have also been observed on mice, suggesting that glutamatergic transmission is implicated in the pathophysiology of affective disorders such as major depression and anxiety (Cryan et al., 2003, Eur. J. Neurosc., 17(11):2409-17). Consequently, any compound able to modulate glutamatergic signalling or function would constitute a promising therapeutic compound for many disorders of the nervous system.

Among abnormalities in glutamatergic transmission, excitotoxicity contributes to neurodegenerative disorders and diseases. Therefore, antiglutamatergic strategies are currently developed especially to fight characteristic symptoms of Amyotrophic Lateral Sclerosis, Parkinson's disease and Alzheimer's Disease. Drugs able to block the transmission of glutamate in a patient in need thereof are referred to as antiglutamatergic or neuroprotective compounds. Riluzole (Rilutek®, Aventis Pharma Inc.), topiramate (Epitomax®, Janssen Cilag) and amantadine (Mantadix®, Bristol-Myers Squibb) are such antiglutamatergic compounds able to modulate, directly or indirectly, glutamate levels. Those compounds seem efficient in the treatment and/or prevention of symptoms linked to various diseases among which epilepsy, Parkinson's disease and Amyotrophic Lateral Sclerosis, but also migraine, depression, bipolar disorders.

In consideration of most of the available antiglutamatergic compounds, which serve as research tools and also as potential therapeutic compounds, we must admit that most of them display a lack of potency and/or selectivity. Accordingly, there is still a need for safe and efficacious antiglutamatergic compounds.

The present invention provides compounds of formula (I): 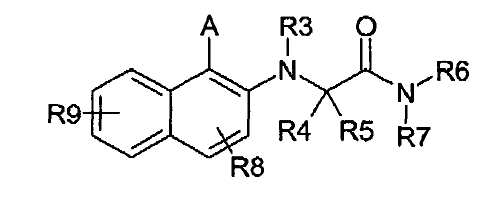 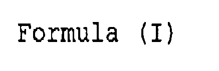 wherein:
**A** is **X-R1** or **N(R1)(R2)** with
   **X** represents oxygen or sulfur;
   **R1** and **R2** are independently from the other hydrogen, alkyl, aryl, or heteroaryl,
   or **R1, R2** and the -N linking them are part of an heterocycloalkyl selected from azetidinyl, pyrrolidinyl, imidazolinyl, pyrolidin-2-one, morpholinyl, thiomorpholinyl, piperidinyl, piperidin-2-one, piperazinyl, N-alkyl-piperazinyl;
   **R1** and **R2** cannot represent simultaneously a hydrogen atom and when **A** is **X-R1, R1** cannot represent a hydrogen atom;
**R3** is hydrogen, alkyl or alkylaryl;
**R4** and **R5** are independently from each other hydrogen, alkyl, aryl, heteroaryl or alkylaryl;
**R6** and **R7** are independently from each other hydrogen, alkyl, aryl or heteroaryl,
or **R6, R7** and the -N linking them are part of an heterocycloalkyl selected from azetidinyl, pyrrolidinyl, imidazolinyl, pyrolidin-2-one, morpholinyl, thiomorpholinyl, piperidinyl, piperidin-2-one, piperazinyl, N-alkyl-piperazinyl;
**R8** and **R9** are independently from each other hydrogen, halogen, alkyl, CN, CF₃, OCF₃, heterocycloalkyl, N(alkyl)₂ or alkoxy;
as well as pharmaceutically acceptable salt thereof, a prodrug of the compound or the salt, or a solvate or hydrate of the compound, the salt or the prodrug.

As used herein, the term **"alkyl"** includes straight or branched chain saturated hydrocarbon residues with 1-8 carbon atoms, such as methyl, ethyl, n-propyl, isopropyl, n-butyl, i-butyl, s-butyl and t-butyl. These alkyls can be further substituted with groups such as COOH, heterocycloalkyl, CF₃, OH, O-alkyl or N-(lower alkyl) (lower alkyl) .
As used herein, the term **"lower alkyl"** includes straight or branched chain saturated hydrocarbon residues with 1-4 carbon atoms, such as methyl, ethyl, n-propyl, isopropyl, n-butyl, i-butyl, s-butyl or t-butyl.
The term **"aryl"** refers to a 6-10 atoms aromatic hydrocarbon ring or fused aromatic hydrocarbon ring system containing at least one unsaturated aromatic ring.
Examples of the term "aryl" are phenyl, naphthyl and 1,2,3,4-tetrahydronaphthyl. These aryls can be further substituted with groups such as halogen, CN, CF₃, OCF₃, lower alkyl, COOH, O-(lower alkyl), S-(lower alkyl), N-(lower alkyl) (lower alkyl) or heterocycloalkyl.
The term **"heteroaryl"** means a 5-10 atoms aromatic ring or fused aromatic rings containing one or more O, S, or N atoms. Examples of heteroaryls include pyridinyl, quinolinyl, isoquinolinyl, pyridazinyl, pyrimidinyl, pyrazinyl, furyl, benzofuryl, thienyl, benzothienyl, pyrrolyl, indolyl, pyrazolyl, indazolyl, oxazolyl, benzoxazolyl, thiazolyl, benzothiazolyl, imidazolyl, benzimidazolyl, and tetrazolyl. These heteroaryls can be further substituted with groups such as halogen, CN, CF₃, OCF₃, lower alkyl, COOH, O-(lower alkyl), S-(lower alkyl), N-(lower alkyl)(lower alkyl) or heterocycloalkyl.
The term **"heterocycloalkyl"** means a 4-6 atoms ring containing one or more O, S, or N atoms. Examples of heterocycloalkyls include azetidinyl, pyrrolidinyl, tetrahydrofuranyl, imidazolinyl, pyrolidin-2-one, morpholinyl, thiomorpholinyl, piperidinyl, piperidin-2-one, piperazinyl, N-alkyl-piperazinyl.
The term **"alkylaryl"** means an alkyl-aryl-group or radical wherein alkyl and aryl have the meanings as defined above. Illustrative examples of an alkylaryl group or radical include benzyl, 2-phenylethyl, 3-phenylpropyl, 4-phenylbutyl, 3-methyl-3-phenylpropyl, 1-naphthylmethyl, 1-naphthylethyl.
The term "alkoxy" means an alkyl-O-group, in which the alkyl group is as previously described.
The term "halogen" refers to bromine, chlorine, fluorine, or iodine.
Pharmaceutically acceptable salts of compounds of formula (I) include salts with inorganic or organic acids, e.g. hydrochloric, hydrobromic, nitric, carbonic, formic, monohydrogencarbonic, phosphoric, monohydrogenphosphoric, dihydrogenphosphoric, perchloric, sulfuric, monohydrogensulfuric, hydroiodic, phosphorous, acetic, lactic, propionic, butyric, isobutyric, palmoic, maleic, glutamic, hydroxymaleic, malonic, benzoic, succinic, glycolic, suberic, fumaric, mandelic, phthalic, salicylic, benzenesulfonic, p-tolylsulfonic, citric, tartaric, methanesulfonic and hydroxynaphthoic acids.
Pharmaceutically acceptable salts of compounds of formula (I) can also include salts with inorganic bases, e.g. alkali metal bases, especially sodium or potassium bases or alkaline-earth metal bases, especially calcium or magnesium bases, or with pharmaceutically acceptable organic bases.

In a particular embodiment, the invention concerns a compound of formula (I), wherein **A** is **X-R1** with:
**R1** is alkyl, aryl or heteroaryl;
**X** and **R3** to **R9** are as defined in formula (I);
as well as pharmaceutically acceptable salt thereof, a prodrug of the compound or the salt, or a solvate or hydrate of the compound, the salt or the prodrug.
In another embodiment, the invention concerns a compound of formula (I), wherein **A** is **N(R1)(R2)** with:
**R1** to **R9** are as defined in formula (I);
as well as pharmaceutically acceptable salt thereof, a prodrug of the compound or the salt, or a solvate or hydrate of the compound, the salt or the prodrug.

In a preferred embodiment, the invention provides compounds of formula (I) wherein **A** is **X-R1** with: **X, R1**, and **R3** to **R7** are as defined in formula (I), and **R8** and **R9** are hydrogen.
In a further preferred embodiment, the invention provides compounds of formula (I) wherein **A** is **N(R1)(R2)** with: **R1** to **R7** are as defined in formula (I), and **R8** and **R9** are hydrogen.

In a specific embodiment, the invention provides compounds of formula (I) wherein **A** is **X-R1** with: **A** is as defined in formula (I) above, and **R3** to **R9** are hydrogen.
In yet a further specific embodiment, the invention relates to compounds of formula (I) wherein **A** is **N(R1)(R2)** with: **A** is as defined in formula (I) above, and **R3** to **R9** are hydrogen.

Preferred compounds of formula (I) according to the invention are:
*2-[1-(2-Morpholin-4-yl-ethoxy)-naphthalen-2-ylamino]-acetamide, hydrochloride* 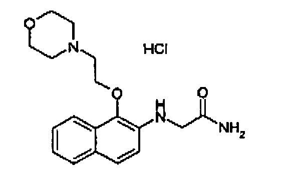
*2-(1-Morpholin-4-yl-naphthalen-2-ylamino)-acetamide, hydrochloride*

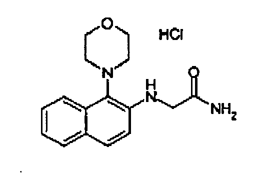

The compounds of general formula (I) and their pharmaceutically acceptable salts can be manufactured according to methods described in the following schemes:

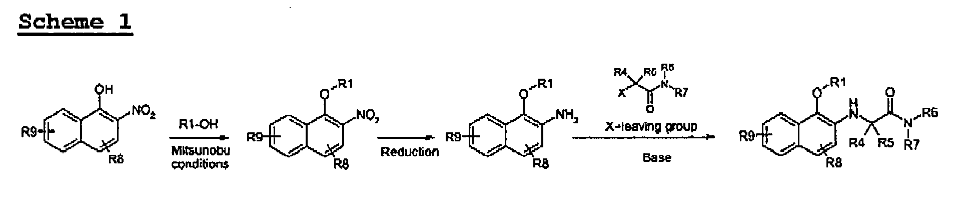

Scheme 1 described route used for synthesis of example 1.

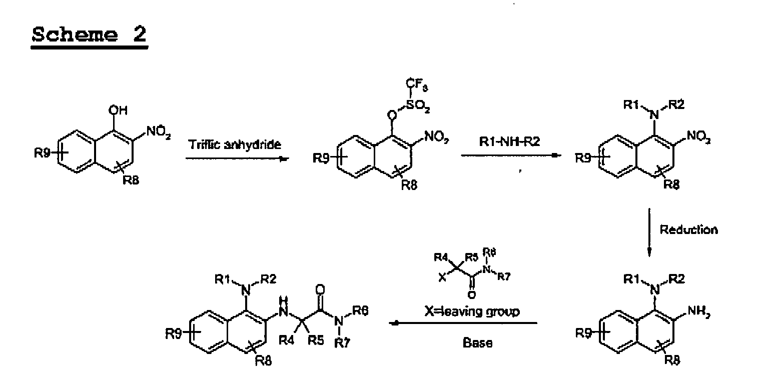

Scheme 2 described route used for synthesis of examples 2 and 3.

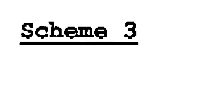

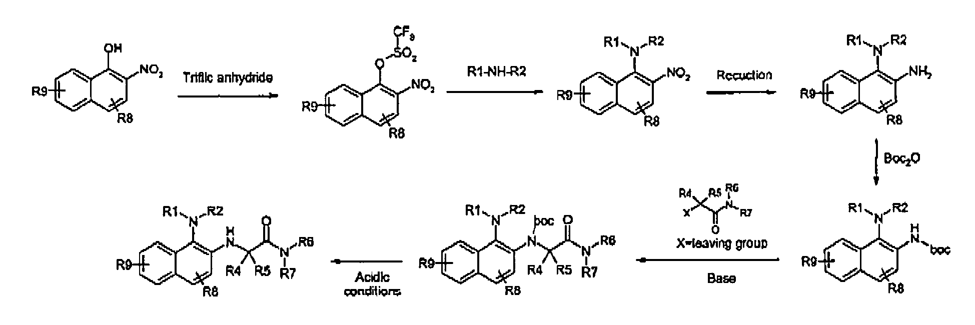

Scheme 3 described route used for synthesis of examples 4 and 5.

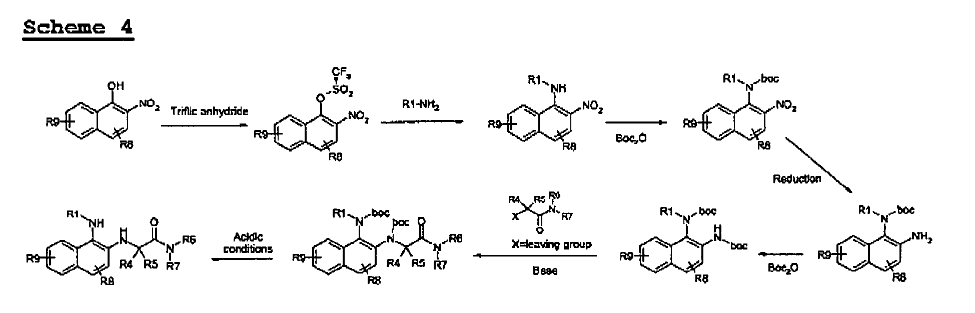

Scheme 4 described route used for synthesis of examples 6 and 7.

The invention further provides the use of a compound of formula (I) in the manufacture of a drug for use in the treatment and/or prophylaxis of conditions and diseases linked to abnormalities in glutamatergic transmission.

The conditions and diseases linked to abnormalities in glutamatergic transmission are epilepsy, including newborn, infantile, childhood and adult syndromes, partial (localization-related) and generalized epilepsies, with partial and generalized, convulsive and non-convulsive seizures, with and without impairment of consciousness, and status epilepticus; Dementias, including dementias of the Alzheimer's type (DAT), Pick's disease, vascular dementias, Lewy-body disease, dementias due to metabolic, toxic and deficiency diseases (including alcoholism, hypothyroidism, and vitamin B12 deficiency), AIDS-dementia complex, Creutzfeld-Jacob disease and atypical subacute spongiform encephalopathy; Parkinsonism and movement disorders, including Parkinson's disease, multiple system atrophy, progressive supranuclear palsy, hepatolenticular degeneration, chorea (including Huntington's disease and hemiballismus), athetosis, dystonias (including spasmodic torticollis, occupational movement disorder, Gilles de la Tourette syndrome), levo-dopa induced dyskinesias, tardive dyskinesias and other drug induced dyskinesias and movement disorders, tremor and myoclonus; Motor neuron disease or amyotrophic lateral sclerosis (ALS); Other neurodegenerative and/or hereditary disorders of the nervous system, including spinocerebrellar degenerations such as Friedrich's ataxia and other hereditary cerebellar ataxias, predominantly spinal muscular atrophies, hereditary neuropathies, and phakomatoses; Disorders of the peripheral nervous system, including trigeminal neuralgia, facial nerve disorders, disorders of the other cranial nerves, nerve root and plexus disorders, mononeuritis such as carpal tunnel syndrome and sciatica, hereditary and idiopathic peripheral neuropathies, inflammatory and toxic neuropathies; Deafness, tinnitus, vertigo. Multiple sclerosis and other demyelinating diseases of the nervous system; Infantile cerebral palsy (spastic), monoplegic, paraplegic or tetraplegic; Hemiplegia and hemiparesis, flaccid or spastic, and other paralytic syndromes; Cerebrovascular disorders, including subarachnoid hemorrhage, intracerebral hemorrhage, occlusion and stenosis of precerebral arteries, occlusion of cerebral arteries including thrombosis and embolism, brain ischemia, stroke, transient ischemic attacks, atherosclerosis, cerebrovascular dementias, aneurysms, cerebral deficits due to cardiac bypass surgery and grafting; Migraine, including classical migraine and variants such as cluster headache; Headache; Myoneural disorders including myasthenia gravis, acute muscle spasms, myopathies including muscular dystrophies, myotonias and familial periodic paralysis; Disorders of the eye and visual pathways , including retinal disorders, and visual disturbances; Intracranial trauma/injury and their sequels; Trauma/injury to nerves and spinal cord and their sequels; Poisoning and toxic effects of non medicinal substances; Accidental poisoning by drugs, medicinal substances and biologicals acting on the central, peripheral and autonomic system; Neurological and psychiatric adverse effects of drugs, medicinal and biological substances; Disturbance of sphincter control and sexual function; Mental disorders usually diagnosed in infancy, childhood or adolescence, including : mental retardation, learning disorders, motor skill disorders, communication disorders, pervasive developmental disorders, attention deficit and disruptive behaviour disorders, feeding and eating disorders, TIC disorders, elimination disorders; Delirium and other cognitive disorders; Substance related disorders including: alcohol-related disorders, nicotine-related disorders, disorders related to cocaine, opioids, cannabis, hallucinogens and other drugs; schizophrenia and other psychotic disorders; Mood disorders, including depressive disorders and bipolar disorders; Anxiety disorders, including panic disorders, phobias, obsessive-compulsive disorders, stress disorders, generalized anxiety disorders; Eating disorders, including anorexia and bulimia; Sleep disorders, including dyssomnias (insomnia, hypersomnia, narcolepsy, breathing related sleep disorder) and parasomnias; Endocrine and metabolic diseases including diabetes, disorders of the endocrine glands, hypoglycaemia; Acute and chronic pain; Nausea and vomiting; Irritable bowel syndrome.

The present invention further provides a pharmaceutical composition comprising a pharmaceutically effective amount of a compound of the formula (I), or pharmaceutically acceptable salts thereof, in combination with a pharmaceutically acceptable carrier, diluent or excipient.

According to the invention, the terms "treatment and/or prophylaxis" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder, improvement of the patient's quality of life.

In another embodiment, the invention provides a pharmaceutical composition for the treatment and/or prophylaxis of a disease or condition selected from epilepsy, dementias (including dementias of the Alzheimer's type, vascular dementias, AIDS-dementia complex, etc...), parkinsonism and movement disorders (including Parkinson's disease, Huntington's disease, dystonias, Gilles de la Tourette syndrome, dyskinesias etc...), motor neuron disease or amyotrophic lateral sclerosis (ALS), other neurodegenerative and/or hereditary disorders of the nervous system (including hereditary cerebellar ataxias and spinal muscular atrophies), disorders of the peripheral nervous system, including trigeminal neuralgia and peripheral neuropathies, multiple sclerosis and other demyelinating diseases of the nervous system, infantile cerebral palsy, spasticity, hemiplegia and hemiparesis, cerebrovascular disorders (including brain ischemia, stroke, transient ischemic attacks, atherosclerosis, etc...), headache, migraine, myoneural disorders (myasthenia gravis, acute muscle spasms, myopathies, etc...), disorders of the eye and visual pathways, intracranial trauma/injury, trauma/injury to nerves and spinal cord, poisoning and toxic effects of non medicinal substances, accidental poisoning by drugs medicinal substances and biologicals, neurological and psychiatric adverse effects of drugs, medicinal and biological substances (including medication-induced movement disorders), disturbance of sphincter control and sexual function, mental disorders usually diagnosed in infancy, childhood or adolescence (including, attention deficit and disruptive behaviour disorders, autism, TIC disorders, etc ...), delirium and other cognitive disorders, substance related disorders (alcohol, nicotine, drugs), schizophrenia and other psychotic disorders, mood disorders (including depressive disorders and bipolar disorders), anxiety disorders, sexual disorders, eating disorders, sleep disorders, endocrine and metabolic diseases (diabetes, hypoglycaemia), acute and chronic pain; nausea and vomiting; irritable bowel syndrome.

The compounds of the invention may be administered in a form suitable for oral use, for example a tablet, capsule, aqueous or oily solution, suspension or emulsion; for topical use including transmucosal and transdermal use, for example a cream, ointment, gel, aqueous solution or suspension, salve, patch or plaster; for nasal use for example a snuff, nasal spray or nasal drops; for vaginal or rectal use, for example a suppository; for administration by inhalation, for example a finely divided powder or a liquid aerosol; for sub-lingual or buccal use, for example a tablet or a capsule; or for parenteral use (including intravenous, subcutaneous, intramuscular, intravascular or infusion), for example a sterile aqueous solution or suspension, or depot injection formulation. In general, the above compositions may be prepared in a conventional manner using well known excipients, using standard techniques, including controlled release technologies, such as gelatin, lipid, gel depot, liposome and/or microcapsule based systems well known to those skilled in the art of pharmacy.
For an oral administration, the compounds of the invention will generally be provided in the form of tablets or capsule or as an aqueous solution or suspension.
Tablets for oral use may include the active ingredient mixed with pharmaceutically acceptable excipients such as inert diluents (such as sodium and calcium carbonate, sodium and calcium phosphate, or lactose), disintegrating agents (such as corn starch or alginic acid), binding agents (starch or gelatin), lubricating agents (magnesium stearate, stearic acid or talc), sweetening agents, flavoring agents, coloring agents and preservatives. If desired, the tablets may be coated with a material such as glyceryl monostearate or glyceryl distearate, to delay absorption in the gastrointestinal tract.
Capsules for oral use include hard gelatin capsules in which the active ingredient is mixed with a solid diluent, and soft gelatin capsules wherein the active ingredient is mixed with water or oil such as peanut oil, liquid paraffin or olive oil.
For intramuscular, intraperitoneal, subcutaneous and intravenous use, the compounds of the invention will generally be provided in sterile aqueous solutions (such as Ringer's solution or isotonic sodium chloride) or suspensions (such as cellulose derivatives, sodium alginate, polyvinylpyrrolidone and gum tragacanth with a wetting agent such as lecithin, and a preservative such as ethyl and n-propyl p-hydroxybenzoate), buffered to an appropriate pH and isotonicity.
Transdermal formulations include membrane permeation systems, multi-laminate adhesive dispersion systems and matrix dispersion systems. Transdermal delivery also includes the use of electrically aided transport and skin penetration enhancers.
The preferred route of administration is the intravenous infusion, preferably over a period of up to seven days, or as an oral formulation, or as an intramuscular injection via a styrette or as a subcutaneous injection.
It will be appreciated that the dosage levels used may vary over quite a wide range depending upon the compound used, the severity of the condition exhibited by the patient and the patient's body weight. However, without commitment to a rigid definition of dosages, it may be stated that a daily dosage of the active ingredient is 100 µg to 800 mg.

Other characteristics and advantages of the invention are given in the following examples in which reference is made to:

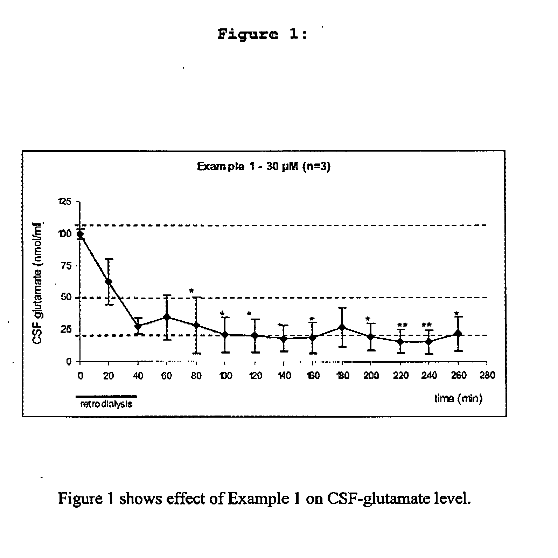

- Figure. 1 which represents the effect of the compound of Example 1 below, on CSF-glutamate level,

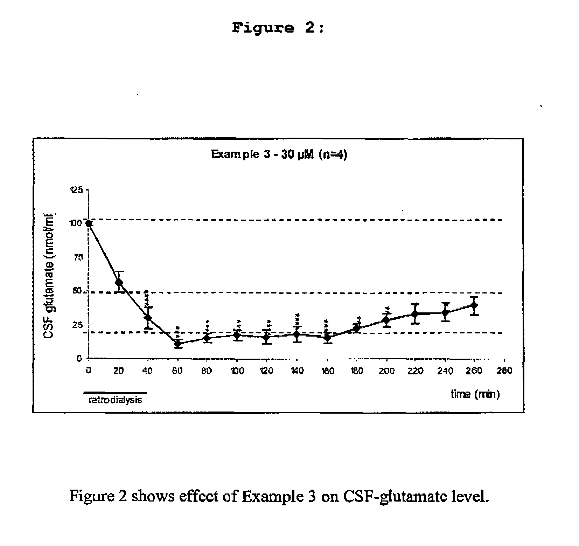

- Figure 2 which represents the effect of the compound of Example 3 below, on CSF-glutamate level.
It will be appreciated that the invention is described by way of example only and modification of detail may be made without departing from the scope of the invention.

### Experimental:

### Preparation A: 4-[2-(2-Nitro-naphthalen-1-yloxy)-ethyl]-morpholine

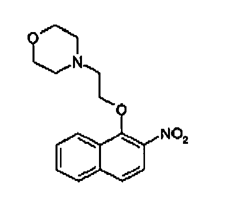

Diisopropyl azodicarboxylate (1.0 equiv) was added dropwise to an ice-cooled solution of 2-nitro-1-naphthol (0.5 g, 2.6 mmol) and triphenylphosphine (1.0 equiv) in toluene (30 mL). The resulting solution was stirred at 0°C for 10 min and 4-(2-hydroxyethyl)morpholine (1.0 equiv) was added. The reaction mixture was allowed to heat to 80°C for three hours. The solvent was evaporated. The residue was dissolved in EtOAc (30 mL) and washed successively with saturated aqueous NH₄Cl solution (2x) and brine. After drying over MgSO₄, the solvent was evaporated to give crude nitronaphthalene. This latter was purified by flash chromatography (cyclohexane/EtOAc 1:1) to afford brown oil in 82% yield.
¹H-NMR (400 MHz, CDCl₃) : 2.61 (br, 4H, CH₂-N), 2.95 (br, 2H, CH₂-N), 3.77 (br, 4H, CH₂-O), 4.37 (br, 2H, CH₂-O), 7.70 (m, 3H, Ar), 7.92-7.93 (m, 2H, Ar), 8.47 (m, 1H, Ar).
M/Z (M+H)⁺ = 303.

### Preparation B: 1-(2-Morpholin-4-yl-ethoxy)-naphthalen-2-ylamine

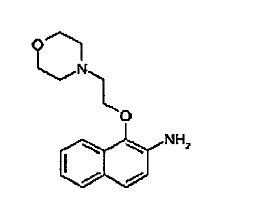

A catalytic amount of Pd/C (5% w/w) was added to a solution of 4-[2-(2-Nitro-naphthalen-1-yloxy)-ethyl]-morpholine (2.9 g, 9.5 mmol) in EtOH/EtOAc (80 mL, 1:1). The solution was stirred under H₂ at r.t. for 18 hours. The mixture was filtered on celite®, rinsed with EtOH/EtOAc (50 mL, 1:1). The filtrate was concentrated to afford a purple solid in 90 % yield.
¹H NMR (400 MHz, CDCl₃): 2.66 (br, 4H, CH₂-N), 2.80 (m, 2H, CH₂-N), 3.81 (t, *J* 4.6 Hz, 4H, CH₂-O), 4.14 (m, 2H, CH₂-O), 4.67 (br, 2H, -NH₂), 7.02 (d, *J* 8.6 Hz, 1H, Ar), 7.25 (m, 1H, Ar), 7.43 (m, 1H, Ar), 7.49 (d, *J* 8.6 Hz, 1H, Ar), 7.72 (m, 1H, Ar), 7.95 (m, 1H, Ar).
M/Z (M+H)⁺ = 273.

### Preparation C: Trifluoro-methanesulfonic acid 2-nitro-naphthalen-1-yl ester

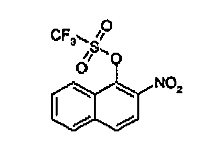

Triflic anhydride (1.5 equiv) was added dropwise to a solution of 2-nitro-1-naphthol (2.24 g, 11.8 mmol) and triethylamine (3 equiv) in DCM (50 mL) at -10°C. The resulting solution was stirred at -10°C for 30 min and then allowed to warm to r.t. The reaction mixture was diluted with CH₂Cl₂ and the organic layer was washed successively with saturated aqueous NH₄Cl solution (2x), water (2x) and brine. After drying over MgSO₄, the solvent was evaporated to afford a brown solid in quantitative yield.
¹H NMR (400 MHz, CDCl₃): 7.82 (m, 2H, Ar), 8.03 (m, 2H, Ar), 8.12 (d, *J* 9.0 Hz, 1H, Ar), 8.32 (m, 1H, Ar).

### Preparation D: Dimethyl-(2-nitro-naphthalen-1-yl)-amine

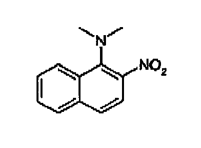

Dimethylamine (3.5 equiv, 2.0 M/THF) was added slowly to a solution of trifluoro-methanesulfonic acid 2-nitro-naphthalen-1-yl ester (0.5 g, 1.56 mmol) in acetonitrile (6 mL). The solution was allowed to heat to 140°C under microwave irradiation for 4 minutes. The resulting mixture was evaporated and purified by flash chromatography (EtOAc/cyclohexane 8:2) to afford an orange oil in quantitative yield.
¹H NMR (400 MHz, CDCl₃): 3.01 (s, 6H, CH₃-N), 7.63 (m, 4H, Ar), 7.87 (m, 1H, Ar), 8.35 (m, 1H, Ar).
M/Z (M+H)⁺ = 217.

### Preparation E: N1,N1-Dimethyl-naphthalene-1,2-diamine

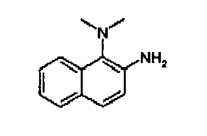

A catalytic amount of Pd/C (5% w/w) was added to a solution of dimethyl-(2-nitro-naphthalen-1-yl)-amine (2.4 g, 11 mmol) in EtOH/EtOAc (50 mL, 1:1). The mixture was stirred under H₂ at r.t. for 18 hours. The mixture was filtered on celite®, rinsed with EtOH/EtOAc (30 mL, 1:1). The filtrate was concentrated to afford a black solid in quantitative yield.
¹H NMR (400 MHz, CDCl₃): 3.06 (s, 6H, CH₃-N), 7.06 (d, *J* 8.6 Hz, 1H, Ar), 7.26 (m, 1H, Ar), 7.44 (m, 1H, Ar), 7.55 (d, *J* 8.6 Hz, 1H, Ar), 7.78 (d, *J* 8.5 Hz, 1H, Ar), 7.89 (d, *J* 8.5 Hz, 1H, Ar).
M/Z (M+H)⁺ = 187.

### Preparation F: 4-(2-Nitro-naphthalen-1-yl)-morpholine

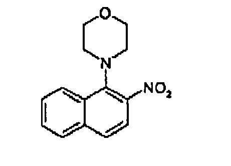

Morpholine (3.5 equiv) was added slowly to a solution of trifluoro-methanesulfonic acid 2-nitro-naphthalen-1-yl ester (0.95 g, 2.96 mmol) in acetonitrile (10 mL). The solution was allowed to heat to 150°C under microwave irradiation for 5 minutes. The resulting mixture was evaporated and purified by flash chromatography (cyclohexane/EtOAc 1:1) to afford an orange oil in quantitative yield.
¹H NMR (400 MHz, CDCl₃): 3.28 (br, 4H, CH₂-N), 3.98 (t, J 4.6 Hz, 4H, CH₂-O), 7.66 (m, 4H, Ar), 7.91 (m, 1H, Ar), 8.42 (m, 1H, Ar).
M/Z (M+H)⁺ = 259.

### Preparation G: 1-Morpholin-4-yl-naphthalen-2-ylamine

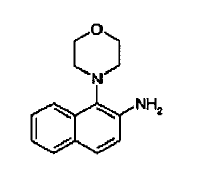

A catalytic amount of Pd/C (5% w/w) was added to a solution of 4-(2-nitro-naphthalen-1-yl)-morpholine (1.7 g, 6.6 mmol) in EtOH/EtOAc (30 mL, 1:1). The solution was stirred under H₂ at r.t. for 18 hours. The mixture was filtered on celite®, rinsed with EtOH/EtOAc (20 mL, 1:1). The filtrate was concentrated to afford a brown solid in 94 % yield.
¹H NMR (400 MHz, CDCl₃): 2.95 (d, J 12.2 Hz, 2H, CH₂-N), 3.81 (m, 4H, CH₂-O), 4.02 (m, 2H, CH₂-N), 7.07 (d, J 8.6 Hz, 1H, Ar), 7.24 (m, 1H, Ar), 7.43 (m, 1H, Ar), 7.57 (d, J 8.6 Hz, 1H, Ar), 7.75 (d, J 8.1 Hz, 1H, Ar), 8.03 (d, J 8.6 Hz, 1H, Ar).
M/Z (M+H) ⁺ = 229.

### Preparation H: 1-Methyl-4-(2-nitro-naphthalen-1-yl)- piperazine

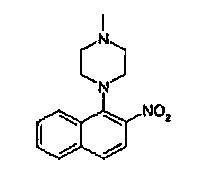

Morpholine (3.5 equiv) was added slowly to a solution of trifluoro-methanesulfonic acid 2-nitro-naphthalen-1-yl ester (0.95 g, 2.96 mmol) in acetonitrile (10 mL). The mixture was allowed to heat to 150°C under microwave irradiation for 5 minutes. The solvent was evaporated and the product was purified by flash chromatography (cyclohexane/EtOAc 6:4) to afford an orange oil in quantitative yield.
¹H NMR (400 MHz, CDCl₃) : 2.45 (s, 3H, CH₃-N), 2.71 (br, 4H, CH₂-N), 3.31 (br, 4H, CH₂-O), 7.63 (m, 4H, Ar), 7.89 (m, 1H, Ar), 8.39 (m, 1H, Ar)
M/Z (M+H)⁺ = 272.

### Preparation I: 1-(4-Methyl-piperazin-1-yl)-naphthalen-2-ylamine

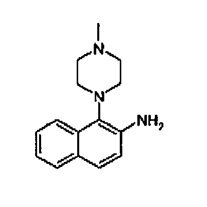

A catalytic amount of Pd/C (5% w/w) was added to a solution of 1-methyl-4-(2-nitro-naphthalen-1-yl)-piperazine (1.52 g, 5.6 mmol) in EtOH/EtOAc (25 mL, 1:1). The solution was stirred under H₂ at r.t. for 18 hours. The mixture was filtered on celite®, rinsed with EtOH/EtOAc (15 mL, 1:1). The filtrate was concentrated to afford a brown solid in 99 % yield.
¹H NMR (400 MHz, CDCl₃): 2.42 (m, 2H, CH₂-N), 2.46 (s, 3H, CH₃-N), 2.90 (d, J 11. 5 Hz, 2H, CH₂-N), 3.04 (d, J 12.1 Hz, 2H, CH₂-N), 3.76 (m, 2H, CH₂-N), 4.43 (br, 2H, -NH₂), 7.04 (d, *J* 8. 7 Hz, 1H, Ar), 7.22 (m, 1H, Ar), 7.38 (m, 1H, Ar), 7.54 (d, *J* 8. 6 Hz, 1H, Ar), 7.73 (d, *J* 8.1 Hz, 1H, Ar), 8.06 (d, *J* 8.6 Hz, 1H, Ar).
M/Z (M+H)⁺ = 242.

### Preparation J: [1-(4-Methyl-piperazin-1-yl)-naphthalen-2-yl]-carbamic acid tert-butyl ester

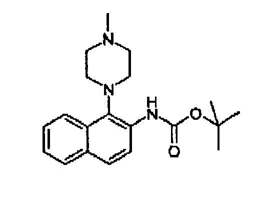

Di-tert-butyl dicarbonate (1.2 equiv) was added to a solution of 1-(4-methyl-piperazin-1-yl)-naphthalen-2-ylamine (0.2 g, 0.83 mmol) in THF (4 mL). The resulting mixture was allowed to heat to 50°C under nitrogen for 18 hours. The solvent was evaporated and the residue was purified by flash chromatography (cyclohexane/EtOAc 1:1) to afford a red solid in 55 % yield.
¹H NMR (400 MHz, CDCl₃): 1.6 (s, 9H, (CH₃)₃-C), 2.38 (m, 2H, CH₂-N), 2.47 (s, 3H, CH₃-N), 2.95 (m, 4H, CH₂-N), 3.85 (m, 2H, CH₂-N), 7.34 (m, 1H, Ar), 7.43 (m, 1H, Ar), 7.71 (d, *J* 9.1 Hz, 1H, Ar), 7.81 (d, *J* 8.8 Hz, 1H, Ar), 8.12 (d, *J* 8.4 Hz, 1H, Ar), 8.41 (m, 2H, Ar, NH)
M/Z (M+H)⁺ = 342.

### Preparation K: Carbamoylmethyl-[1-(4-methyl-piperazin-1-yl)-naphthalen-2-yl]-carbamic acid tert-butyl ester

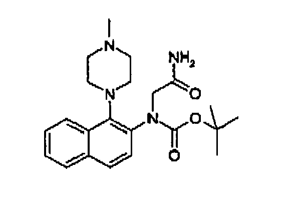

Sodium hydride (2 equiv) was added to a solution of carbamoylmethyl-[1-(4-methyl-piperazin--1-yl)-naphthalen-2-yl]-carbamic acid tert-butyl ester (150 mg, 0.44 mmol) in anhydrous DMF (3 mL) at - 10°C under nitrogen. After 10 minutes the bromoacetamide (3 equiv) was added. The resulting mixture was allowed to warm to r.t., stir for 18 hours and then quench with saturated aqueous NH₄Cl solution (5 mL). The pH was adjusted to 10-11 using NaOH 6N. The mixture was extracted with EtOAC (20 mL) and successively washed with water and brine. After drying over MgSO₄, the solvent was evaporated to give crude naphthalene. This latter was purified by flash chromatography (5% MeOH/EtOAc) to afford colourless oil in 47 % yield.
¹H NMR (400 MHz, CDCl₃): 1.64 (s, 9H, (CH₃)₃-C), 2.44 (s, 3H, CH₃-N), 2.5-2.7 (m, 4H, CH₂-N), 3.27 (m, 2H, CH₂-N), 3.42 (m, 2H, CH₂-N), 3.61 (d, J 14.9 Hz, 1H, CH₂-CO), 4.70 (d, J 15.8 Hz, 1H, CH₂-CO), 7.32 (m, 1H, Ar), 7.51 (m, 2H, Ar), 7.59 (m, 1H, Ar), 7.83 (m, 1H, Ar), 8.28 (m, 1H, Ar).
M/Z (M+H) = 399.

### Preparation L: (1-Carbamoyl-ethyl)-[1-(4-methyl-piperazin-1-yl)-naphthalen-2-yl]-carbamic acid tert-butyl ester

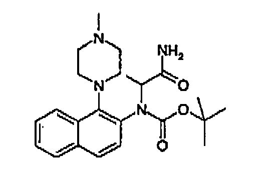

Sodium hydride (2 equiv) was added to a solution of carbamoylmethyl-[1-(4-methyl-piperazin-1-yl)-naphthalen-2-yl]-carbamic acid tert-butyl ester (300 mg, 0.88 mmol) in anhydrous DMF (5 mL) at - 10°C under nitrogen. After 10 minutes, potassium iodide (0.5 equiv) and the 2-bromo-propionamide (5 equiv) were added The resulting mixture was allowed to warm to r.t., stir for 30 minutes and then quench with saturated aqueous NH₁Cl solution (5 mL). The pH was adjusted to 10-11 using NaOH 6N. The mixture was extracted with ethyl acetate (30 mL) and successively washed with water and brine. After drying over MgSO₄, the solvent was evaporated to give the crude naphthalene. This latter was purified by flash chromatography (10 % MeOH/EtOAc) to afford white solid in 33 % yield.
M/Z (M+H)⁺ = 413.

### Preparation M: (2-Nitro-naphthalen-1-yl)-(2-piperidin-1-yl-ethyl)-amine

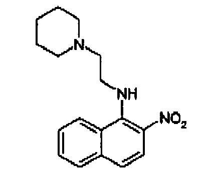

2-Piperidin-1-yl-ethylamine (3.5 equiv) was added slowly to a solution of trifluoro-methanesulfonic acid 2-nitro-naphthalen-1-yl ester (1 g, 3.11 mmol) in acetonitrile (8 mL). The resulting mixture was allowed to heat to 150°C under microwave irradiation for 5 minutes. The solvent was evaporated and the product was purified by flash chromatography (cyclohaxen/EtOAc 8:2) to afford an orange oil in quantitative yield.
¹H NMR (400 MHz, CDCl₃) : 1.45 (m, 2H, CH₂-C), 1.62 (m, 4H, CH₂-C), 2.44 (br, 4H, CH₂-N), 2.54 (t, J 5. 8 Hz, 2H, CH₂-N), 3.81 (m, 2H, CH₂-N), 7.12 (d, J 9.3 Hz, 1H, Ar), 7.48 (m, 1H, Ar), 7.61 (m, 1H, Ar), 7.76 (d, J 8.1 Hz, 1H, Ar), 8.1 (d, J 9.2 Hz, 1H, Ar), 8.35 (d, J 8.6 Hz, 1H, Ar), 9.25 (br, 1H, NH).
M/Z (M+H)⁺ = 300.

### Preparation N: N1-(2-Piperidin-1-yl-ethyl)-naphthalene-1,2-diamine

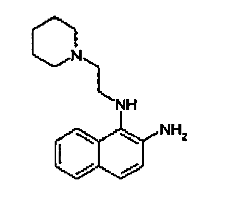

A catalytic amount of Pd/C (5% w/w) was added to a solution of (2-nitro-naphthalen-1-yl)-(2-piperidin-1-yl-ethyl)-amine (770 mg, 2.58 mmol) in EtOH/EtOAc (20 mL, 1:1). The mixture was stirred under H₂ at r.t. for 18 hours, filtered on celite® and rinsed with EtOH/EtOAc (15 mL, 1:1). The filtrate was concentrated to afford a brown solid in 90 % yield.
¹H NMR (400 MHz, CDCl₃) : 1.54 (br, 2H, CH₂-C), 1.73 (m, 4H, CH₂-C), 2.50 (m, 2H, CH₂-N), 2.56 (br, 4H, CH₂-N), 3.23 (t, *J* 5.4 Hz, 2H, CH₂-N), 7.03 (d, *J* 8.6 Hz, 1H, Ar), 7.24 (m, 1H, Ar), 7.44 (m, 1H, Ar), 7.47 (d, *J* 8.5 Hz, 1H, Ar), 7.73 (d, *J* 8.1 Hz, 1H, Ar), 8.01 (d, *J* 8.4 Hz, 1H, Ar).
M/Z (M+H)⁺ = 270.

### Preparation O: (2-tert-Butoxycarbonylamino-naphthalen-1-yl)-(2-piperidin-1-yl-ethyl)-carbamic acid tert-butyl ester

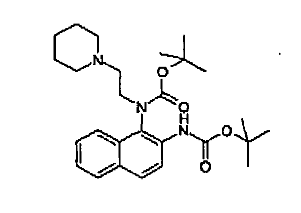

Di-tert-butyl dicarbonate (5 equiv) was added to a solution of *N*-1-(2-piperidin-1-yl-ethyl)-naphthalene-1,2-diamine (0.62 g, 2.3 mmol) in THF (12 mL). The resulting mixture was allowed to heat to 50°C under nitrogen for 18 hours. The solvent was evaporated. The residue was purified by flash chromatography (cyclohexane/EtOAc 9:1) to afford brown oil in quantitative yield.
M/Z (M+H)⁺ = 470.

### Preparation P: [2-(tert-Butoxycarbonyl-diethylcarbamoylmethyl-amino)-naphthalen-1-yl]-(2-piperidin-1-yl-ethyl)-carbamic acid tert-butyl ester

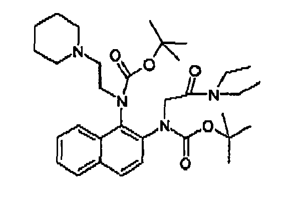

Sodium hydride (2 equiv) was added to a solution of (2-tert-butoxycarbonylamino-naphthalen-1-yl)-(2-piperidin-1-yl-ethyl)-carbamic acid tert-butyl ester (270 mg, 0.57 mmol) in anhydrous DMF (4 mL) at - 10°C under nitrogen. After 10 minutes, potassium iodide (0.5 equiv) and the 2-chloro-acetamide (2.5 equiv) were added. The resulting mixture was allowed to warm to r.t., stir for 18 hours and then quench with saturated aqueous NH₄Cl solution (5 mL). The pH was then adjusted to 10-11 using NaOH 6N. The mixture was extracted with EtOAc (20 mL) and successively washed with water and brine. After drying over MgSO₄, the solvent was evaporated to afford the crude naphthalene. This latter was purified by flash chromatography (cyclohexane/EtOAc 95:5) to afford off-white solid in 75 % yield.
M/Z (M+H)⁺ = 583.

### Preparation Q: (2-Morpholin-4-yl-ethyl)-(2-nitro-naphthalen-1-yl)-amine

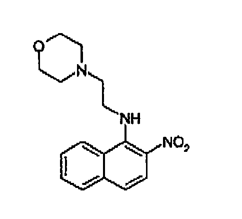

4-(2-Aminoethyl)morpholine (2.5 equiv) was added slowly to a solution of trifluoro-methanesulfonic acid 2-nitro-naphthalen-1-yl ester (1 g, 3.1 mmol) in acetonitrile (8 mL). The solution was allowed to heat to 150°C under microwave irradiation for 5 minutes. The resulting mixture was evaporated and purified by flash chromatography (cyclohexane/CH₂Cl₂ 1:1) to afford an orange oil in quantitative yield.
M/Z (M+H)⁺ = 302.

### Preparation R: N1-(2-Marpholin-4-yl-ethyl)-naphthalene-1,2-diamine

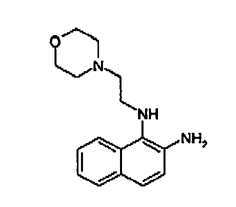

A catalytic amount of Pd/C (5% w/w) was added to a solution of (2-morpholin-4-yl-ethyl)-(2-nitro-naphthalen-1-yl)-amine (3.25 g, 10.9 mmol) in EtOH/EtOAc (90 mL, 1:1). The mixture was stirred under H₂ at r.t. for 18 hours, filtered on celite® and rinsed with EtOH/EtOAc (60 mL, 1:1). The filtrate was concentrated to afford a black solid in 90 % yield.
¹H NMR (400 MHz, CDCl₃) : 2.56 (m, 2H, CH₂-N), 2.61 (br, 4H, CH₂-N), 3.20 (t, J 5.4 Hz, 2H, CH₂-N), 3.86 (t, *J* 4.7 Hz, 4H, CH₂-O), 7.04 (d, *J* 8.6 Hz, 1H, Ar), 7.25 (m, 1H, Ar), 7.44 (m, 1H, Ar), 7.49 (d, *J* 8.6 Hz, 1H, Ar), 7.74 (d, *J* 8.1 Hz, 1H, Ar), 7.97 7 (d, *J* 8.1 Hz, 1H, Ar).
M/Z (M+H)⁺ = 272.

### Preparation S: (2-tert-Butoxycarbonylamino-naphthalen-1-yl)-(2-morpholin-4-yl-ethyl)-carbamic acid tert-butyl ester

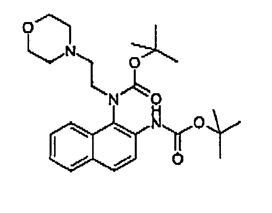

Di-*tert*-butyl dicarbonate (5 equiv) was added to a solution of N1-(2-morpholin-4-yl-ethyl)-naphthalene-1,2-diamine (1.7 g, 6.2 mmol) in THF (50 mL). The resulting mixture was allowed to heat to 50°C under nitrogen for 18 hours. The solvent was evaporated and the residue was purified by flash chromatography (cyclohexane/EtOAc 9:1) to afford purple solid in 72 % yield.
¹H NMR (400 MHz, CDCl₃): 1.45 (s, 9H, (CH₃)₃-C), 1.59 (s, 9H, (CH₃)₃-C), 2.34 (m, 4H, CH₂-N), 2.82 (br, 2H, CH₂-N), 3.21 (dt, *J* 3.8 Hz, *J* 14.8 Hz, 1H, CH₂-N), 3.82 (m, 2H, CH₂-O), 3.95 (m, 2H, CH₂-O), 4.76 (m, 1H, CH₂-N), 7.44 (m, 1H, Ar), 7.52 (m, 1H, Ar), 7.69 (d, *J* 8.5 Hz, 1H, Ar), 7.80 (d, *J* 9.0 Hz, 1H, Ar), 7.85 (d, *J* 7.7 Hz, 1H, Ar), 8.02 (d, *J* 9.0 Hz, 1H, Ar), 9.95 (s, 1H, NH).
M/Z (M+H)⁺ = 472.

### Preparation T: [2-(tert-Butoxycarbonyl-diethylcarbamoylmethyl-amino)-naphthalen-1-yl]-(2-morpholin-4-yl-ethyl)-carbamic acid tert-butyl ester

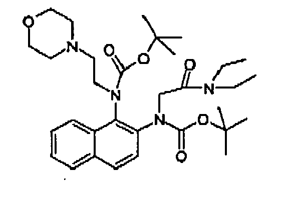

Sodium hydride (2 equiv) was added to a solution of (2-tert-butoxycarbonylamino-naphthalen-1-yl)-(2-morpholin-4-yl-ethyl)-carbamic acid tert-butyl ester (0.5 g, 0.57 mmol) in anhydrous DMF (5 mL) at - 10°C under nitrogen. After 10 minutes, potassium iodide (0.5 equiv) and 2-chloro-acetamide (2.5 equiv) were added. The solution was allowed to warm to r.t. and stir for 18 hours. The resulting mixture was quenched with saturated aqueous NH₄Cl solution (5 mL). The pH was then adjusted to 10-11 using NaOH 6N. The mixture was extracted with EtOAc (30 mL) and successively washed with water and brine. After drying over MgSO₄, the solvent was evaporated to give the crude naphthalene. This latter was purified by flash chromatography (cyclohexane/EtOAc 6:4) to afford off-white solid in 92 % yield.
M/Z (M+H)⁺ = 585.

### Preparation U: Benzyl-(1-morpholin-4-yl-naphthalen-2-yl)-amine

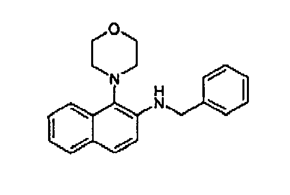

Benzaldehyde (1 equiv) was added to a solution of 1-Morpholin-4-yl-naphthalen-2-ylamine (0.45 g, 1.97 mmol) in ethanol (5 mL). The resulting mixture was allowed to heat to 80°C for 18 hours. Solvent were evaporated. The residue was solubilized in CHCl₃/iPrOH (15 mL, 3:1) and NaBH₄ (2.6 equiv) was added at 0°C in 3 portions. After 10 minutes at 0°C, the solution was allowed to warm to r.t. and stir for 18 hours. Solvents were evaporated. The residue was hydrolysed with NaOH 1N (15 mL) and extracted with EtOAc (20 mL). The organic layer was successively washed with water and brine. After drying over MgSO₄, the solvent was evaporated to give crude benzyl naphthalene. This latter was purified by flash chromatography (cyclohexane/EtOAc 9:1) to afford a red solid in 10 % yield.
¹H NMR (400 MHz, CDCl₃) : 2.82 (m, 2H, CH₂-N), 3.74-3.90 (m, 4H, CH₂-O), 4.02 (s, 2H, CH₂-N), 4.55 (s, 2H, NH-CH₂-Φ), 7.09 (d, *J* 7.6 Hz, 1H, Ar), 7.20 (m, 1H, Ar), 7.36 (m, 5H, Ar), 7.41 (m, 1H, Ar), 7.61 (d, *J* 8.8 Hz, 1H, Ar), 7.73 (m, 1H, Ar), 7.99 (d, *J* 8.2 Hz, 1H, Ar).
M/Z (M+H)⁺ = 319.

The following examples of compounds of formula (I) were obtained using above described routes of synthesis.

### Example 1: 2-[1-(2-Morpholin-4-yl-ethoxy)-naphthalen-2-ylamino]-acetamide, hydrochloride

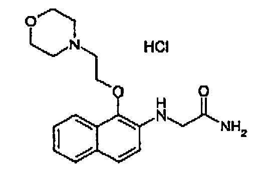

2-Bromoacetamide (1.05 equiv), potassium carbonate (1.5 equiv) and sodium iodide (0.1 equiv) were added at r.t. to a solution of 1-(2-Morpholin-4-yl-ethoxy)-naphthalen-2-ylamine (50 mg, 0.18 mmol) in acetonitrile (3 mL). The resulting mixture was allowed to heat to 80°C for 18 hours. water (10 mL) and DCM (10 mL) were added to the mixture. The organic layer was washed with water (1x) and brine (2x). After drying over MgSO₄, the solvent was evaporated to afford the crude compound. This latter was purified by flash chromatography (100% EtOAc to 10% MeOH/EtOAC) to afford brown oil in 63 % yield. A 2.0 M solution of HCl in ether (10 equiv) was added to a solution of this latter in 0.5 mL of methanol to afford the HCl salt as an brown hygroscopic solid.
¹H NMR (400 MHz, CD₃OD) : 3.38 (s, 2H, CH₂-N), 3.86 (t, *J* 4.9 Hz, 4H, CH₂-N), 4.0-4.1 (m, 4H, CH₂-O), 4.14 (s, 2H, NH-CH₂-CO), 4.57 (t, *J* 4.8 Hz, 2H, CH₂-CO), 7.31 (d, *J* 9.3 Hz, 1H, Ar), 7.45 (m, 1H, Ar), 7.60 (m, 1H, Ar), 7.60 (m, 1H, Ar), 7.77 (d, *J* 8.8 Hz, 1H, Ar), 7.90 (d, *J* 7.8 Hz, 1H, Ar), 8.02 (d, *J* 9.3 Hz, 1H, Ar).
M/Z (M+H)⁺ = 330.

### Example 2: 2-(1-Dimethylamino-naphthalen-2-ylamino)-acetamide, hydrochloride

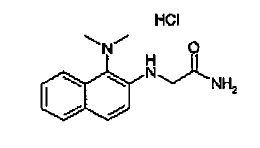

Example 2 was prepared following the procedure described for example 1. The hygroscopic pink solid was obtained in 66% yield.
¹H NMR (400 MHz, DMSO): 3.26 (br, 6H, CH₃-N), 3.96 (s, 2H, NH-CH₂-CO), 7.16 (d, J 9 Hz, 1H, Ar), 7.27 (br, 1H, NH), 7.36 (m, 1H, Ar), 7.55 (m, 2H, Ar), 7.52 (br, 1H, NH), 7.72 (br, 1H, NH), 7.87 (m, 2H, Ar), 8.15 (br, 1H, NH).
M/Z (M+H)⁺ = 244.

### Example 3: 2-(1-Morpholin-4-yl-naphthalen-2-ylamino)-acetamide, hydrochloride

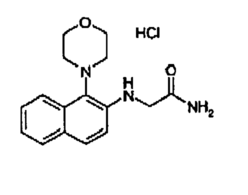

Example 3 was prepared following the procedure described for example 1. The off-white solid was obtained in 54 % yield.
¹H NMR (400 MHz, DMSO): 2.86 (m, 2H, CH₂-N), 3.62 (m, 2H, CH₂-N), 3.86 (m, 4H, CH₂-O), 3.91 (s, 2H, NH-CH₂-CO), 7.13 (d, *J* 8.8 Hz, 1H, Ar), 7.26 (m, 1H, Ar), 7. 32 (br, 1H, NH), 7.45 (m, 1H, Ar), 7.74 (d, *J* 8.8 Hz, 1H, Ar), 7.82 (d, *J* 8. 0 Hz, 1H, Ar), 8. 3 (d, *J* 8.6 Hz, 1H, Ar).
M/Z (M+H)⁺ = 286.
mp = 210°C.

### Example 4: 2-[1-(4-Methyl-piperazin-1-yl)-naphthalen-2-ylamino]-acetamide, hydrochloride

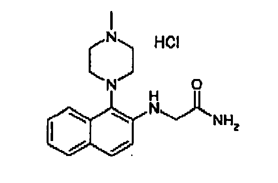

A 2.0 M solution of HCl in ether (5 equiv) was added to a solution of (1-Carbamoyl-ethyly-[1-(4-methyl-piperazin-1-yl)-naphthalen-2-yl]-carbamic acid tert-butyl ester (73 mg, 0.18 mmol) in methanol (1 mL). The resulting mixture was allowed to stir at r.t. for 18 hours. The precipitate obtained was filtered and washed with ether to afford an off-white solid in 93%.
¹H NMR (400 MHz, DMSO) : 2.86 (d, *J* 4.7 Hz, 3H, CH₃-N), 3.02 (m, 2H, CH₂-N), 3.45 (br, 4H, CH₂-O), 3.80 (s, 2H, NH-CH₂-CO), 4.03 (m, 2H, CH₂-N), 6. 93 (d, *J* 8.9 Hz, 1H, Ar), 7.14-7.18 (m, 2H, Ar, NH), 7.37-7.41 (m, 2H, Ar, NH), 7.67 (d, *J* 8.8 Hz, 1H, Ar), 7.75 (d, *J* 7.7 Hz, 1H, Ar), 8.83 (d, *J* 8.7 Hz, 1H, Ar).
M/Z (M+H)⁺ = 299.
mp = 237-239°C.

### Example 5: 2-[1-(4-Methyl-piperazin-1-yl)-naphthalen-2-ylamino]-propionamide, hydrochloride

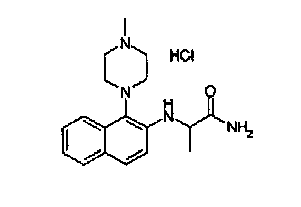

A 2.0 M solution of HCl in ether (5 equiv) was added to a solution of (1-Carbamoyl-ethyl)-[1-(4-methyl-piperazin-1-yl)-naphthalen-2-yl]-carbamic acid tert-butyl ester (41 mg, 0.1 mmol) in methanol (0.5 mL). The resulting mixture was allowed to stirred at r.t. for 18 hours. Methanol was evaporated. The residue was hydrolysed by NaOH 1N (5 mL) and extracted with EtOAc (5 mL) and The organic layer was washed with water (2x) and brine (1x). After drying with MgSO₄, the crude compound was purified by preparative HPLC to afford the desire compound in 44 %. A 2.0 M solution of HCl in ether (10 equiv) was added to a solution of this latter in 0.5 mL of methanol to afford the HCl salt as an off-white solid.
¹H NMR (400 MHz, DMSO): 1.44 (d, *J* 6.8 Hz, 3H, CH₃-C), 2.92 (m, 1H, CH₂-N), 3.04 (m, 1H, CH₂-N), 3.34 (m, 2H, CH₂-N), 3.49 (m, 2H, CH₂-N), 4.10 (m, 3H, NH-CH-CO, CH₂-N), 7.06 (d, *J* 8.9 Hz, 1H, Ar), 7.10 (br, 1H, NH), 7.21 (m, 1H, Ar), 7.41 (m, 1H, Ar), 7.61 (br, 1H, NH), 7.69 (d, *J* 8.8 Hz, 1H, Ar), 7.78(d, *J* 8.1 Hz, 1H, Ar), 7.95 (d, *J* 8.6 Hz, 1H, Ar), 11.42 (br, 1H, NH).
M/Z (M+H)⁺ = 313.
mp = 215-217°C.

### Example 6: N,N-Diethyl-2-[1-(2-piperidin-1-yl-ethylamino)-naphthalen-2-ylamino]-acetamide, hydrochloride

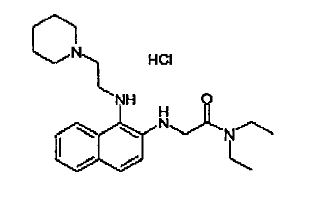

Example 6 was prepared following the procedure described for example 5. The free base was purified by flash chromatography (cyclohexane/EtOAc 2:8) to afford the desire compound in 43 %. A 2.0 M solution of HCl in ether (10 equiv) was added to a solution of this latter in 0.5 mL of methanol to afford the HCl salt as an hygroscopic off-white solid.
¹H NMR (400 MHz, DMSO): 1.05 (t, *J* 7.1 Hz, 3H, CH₃-CH₂-N), 1.18 (t, *J* 7.1 Hz, 3H, CH₃-CH₂-N) , 1.38-1. 48 (br, 2H, CH₂-C), 1.80 (br, 4H, CH₂-C) , 2.96 (br, 2H, CH₂-N) , 3.33 (q, *J* 7.1 Hz, 2H, CH₃-CH₇-N) 3.37-3.43 (m, 6H, CH₂-N, CH₃-CH₂-N) , 3.49 (br, 2H, CH₂-N), 4.17 (s, 2H, NH-CH₂-CO), 7.19 (d, *J* 8.96 Hz, 1H, Ar), 7.24 (m, 1H, Ar), 7.46(m, 1H, Ar), 7.67 (d, *J* 8.8 Hz, 1H, Ar), 7.78 (d, *J* 8.1 Hz, 1H, Ar), 8.11 (d, *J* 8.5 Hz, 1H, Ar) .
M/Z (M+H)⁺ - 383.

### Example 7: N,N-Diethyl-2-[1-(2-morpholin-4-yl-ethylamino)-naphthalen-2-ylamino]-acetamide, hydrochloride

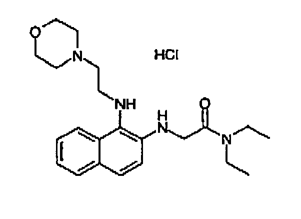

Example 7 was prepared following the procedure described for example 5. The free base was purified by flash chromatography (2 % MeOH/EtOAc) to afford the desire compound in 43 %. A 2.0 M solution of HCl in ether (10 equiv) was added to a solution of this latter in 0.5 mL of methanol to afford the HCl salt as an hygroscopic off-white solid.
¹H NMR (400 MHz, DMSO): 1.05 (t, *J* 7.1 Hz, 3H, CH₃-CH₂-N), 1.18 (t, *J* 7.1 Hz, 3H, CH₃-CH₂-N) , 3.33 (q, *J* 7.1 Hz, 2H, CH₃-CH₂-N), 3.41 (q, *J* 7.1 Hz, 2H, CH₃-CH₂-N), 3.40-3.52 (m, 8H, CH₂-N), 3.93 (m, 4H, CH₂-O), 4.19 (s, 2H, NH-CH₂-CO), 7.21 (d, *J* 8.9 Hz, 1H, Ar), 7.26 (m, 1H, Ar), 7.47 (m, 1H, Ar), 7.69 (d, *J* 8.9 Hz, 1H, Ar), 7.79 (d, *J* 8.1 Hz, 1H, Ar), 8.13 (d, *J* 8.5 Hz, 1H, Ar).
M/Z (M+H)⁺ = 385.

### Example 8: 2-[Benzyl-(1-morpholin-4-yl-naphthalen-2-yl)-amino]-acetamide, hydrochloride

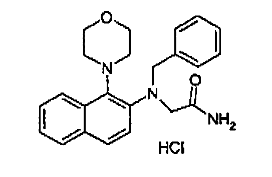

Example 8 was prepared following the procedure described for example 1. The free base was purified by flash chromatography (2 % MeOH/EtOAc) to afford the desire compound in 45 %. A 2.0 M solution of HCl in ether (10 equiv) was added to a solution of this latter in 0.5 mL of methanol to afford the HCl salt as an off-white solid.
¹H NMR (400 MHz, DMSO, 85°C) : 3.00 (under H₂O, 2H, CH₂-N), 3.16 (m, 2H, CH₂-N), 3.80-3.85 (m, 6H, N-CH₂-CO, CH₂-O), 4.50 (s, 2H, N-CH₂-Φ), 7.25 (m, 5H, Ar), 7.41 (m, 1H, Ar), 7.49 (m, 1H, Ar), 7.56 (m, 1H, Ar), 7.69 (m, 1H, Ar), 7.84 (m, 1H, Ar), 8.19 (d, *J* 9.0 Hz, 1H, Ar).
M/Z (M+H)⁺ = 376.
mp = 204-205°C.

### Example 9: Effect of examples 1 and 3 on CSF-glutamate level

For this example, intracerebroventricular retrodialysis was performed on rats according to techniques known to those skilled in the art. The analysis of midrialysates was performed according to Israel and collaborators' publication (Neurochem Int 22(1): 53-58, 1993). Procedure is described below:

### Intracerebroventricular retrodialysis in rats:

Surgery was performed on adult male Sprague-Dawley rats weighing 330-340g at the time of surgery. Following induction of anaesthesia with intraperitoneal administration of ketamine/xylazine, animals were secured in a stereotaxic frame in the flat-skull position at a constant setting of -3.3mm between ear and incisor bars. After defining the "stereotaxic zero" in accordance with Paxinos and Watson's atlas (*1986*), a microdialysis guide canula was directed towards the right lateral ventricle with the coordinates from Bregma: anteroposterior -0.8mm, mediolateral +1.5mm, and dorsoventral (DV) -1.5mm (CMA12 probe: 2mm membrane) (Khandewal et al., J Neurosci Methods 133(1-2): 181-189, 2004).
The guide cannula was fixed to the skull using dental cement and three stainless steel screws. The assembly was protected by a tube (screw-cap tubes Eppendorf).
A plastic collar allowed each animal to be tethered to a Swivel assembly of the CMA awake animal system.
Following surgery, rats were individually housed and were provided food and water *ad libitum.* Animals were allowed 3-4 days of post-operative recovery time.

### In vivo microdialysis

Dialysis probes (CMA12/2mm, 500µm membrane outside diameter) were purchased from CMA/Microdialysis (Stockholm, Sweden) and were equilibrated according to manufacturer's specifications the day before implantation. All experiments were performed between 9.00 am and 6.00 pm. The prepared probe was implanted via the guide cannula into the right lateral ventricle and perfused with artificial cerebrospinal fluid (aCSF: 145mM NaCl, 2.70mM KCl, 1.20mM CaCl₂, 1.0mM MgCl₂, 0.45mM NaH₂PO4 and 2.33mM Na₂HPO₄, pH 7.4) at a flow rate of 1µL/min.
After the probe was inserted into the animal, a 1h stabilization period was allowed after which time dialysate sampling was conducted in the unrestrained rat. For the next 2h, fractions were then collected at 20min intervals into plastic vials in order to estimate the physiological release of the intraventricular CSF glutamate content. Baseline conditions were reached when 3 successive effluent fractions gave similar glutamate contents. Animals were then treated for 40min by retrodialysis without stopping perfusion using 30µM solution of the test compound and dialysis samples were still collected every 20min. After the retrodialysis, dialysis samples were collected for a total period of 4h. The collected fractions (20µL) were immediately, transferred into liquid nitrogen (-180°C) and then kept frozen (-80°C) until further analysis.

### Histology

At the end of each experiment, rats were euthanized and brains were quickly removed and stored in buffered formalin (30% (v/v) formaldehyde/water) for 24h. The correct placement of the microdialysis probe was checked by examination of the coronal sections under a stereomicroscope.
Animals with incorrect probe placement were excluded from the study.

### Analysis of microdialysates

Samples were subsequently analysed for glutamate determination using an enzymatic assay (Israël et al., 1993). The procedure is based on the oxidation of L-glutamate dehydrogenase with subsequent NADH production, the NADH being continuously evaluated using the photobacterium chemiluminescent reaction at 460-490nm.
Microdialysates were extemporaneously thawed and maintained on ice. In a 96-well plate samples were diluted (1:2.5 or 1:5; final volume: 10µL) in assay buffer (50mM NaH₂PO₄ / Na₂HPO₄, pH=7.1-7.2) and the final volume was adjusted to 250µL with assay buffer.
The final enzymatic mix was prepared by adding 550µL of freshly prepared 0.05% decanal (0.05% decanal in 50mM NaH₂PO₄ / Na₂HPO₄, pH=7.4) to 5560µL of enzymatic mix (348U/mL GIDH, 3.59U/mL NAD(P)H:FMN oxidoreductase, 3.59mg/mL Luciferase, 2.98mg/mL β-NAD prepared in 0.2M tris pH=8.0, 5.6µg/mL FMN, and which activity was tested (50µL sample) using a Multiway Analyser (Statice, Besançon, France)) and diluted in assay buffer (1:45). The final enzymatic solution was kept at RT for 15-20min before using in order to allow signal stabilization.
40µL of final containing decanal was added to samples allowing a light emission directly proportional to the quantity of glutamate in each sample. The luminescence was read at 460-490nm.
The assay was performed on a luminescence imaging system with charge coupled device camera CyBi^{™}-Lumax D, using flash-luminescence readout.
The test samples were determined from a standard curve (0 to 25pmol of a standard solution of 1M glutamic acid in osmosed water) prepared in assay buffer in the same conditions. If samples were not enough concentrated, a second run was performed using a standard curve from 0 to 7.5pmol.

### Data analysis

The basal glutamate level for each rat was estimated by averaging the last 3 samples collected before administration of the drug. In all figures, glutamate levels were expressed as a percentage (mean ± SEM; SEM = standard error of the mean) of the baseline arbitrarily set at 100%.

## Claims

1. A compound of formula (I): 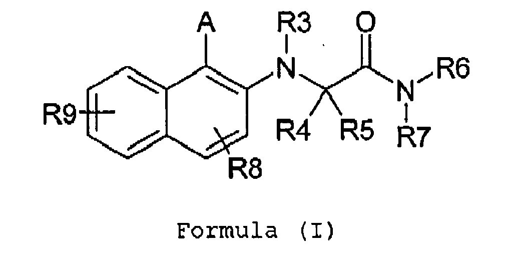 wherein:
**A** is **x-R1** or **N(R1) (R2)** with
**X** represents oxygen or sulfur;
**R1** and **R2** are independently from the other hydrogen, alkyl, aryl, or heteroaryl,
or **R1, R2** and the -N linking them are part of an heterocycloalkyl selected from azetidinyl, pyrrolidinyl, imidazolinyl, pyrolidin-2-one, morpholinyl, thiomorpholinyl, piperidinyl, piperidin-2-one, piperazinyl, N-alkyl-piperazinyl;
**R1** and **R2** cannot represent simultaneously a hydrogen atom and when **A** is **X-R1, R1** cannot represent a hydrogen atom;
**R3** is hydrogen, alkyl or alkylaryl;
**R4** and **R5** are independently from each other hydrogen, alkyl, aryl, heteroaryl or alkylaryl;
**R6** and **R7** are independently from each other hydrogen, alkyl, aryl or heteroaryl,
or **R6, R7** and the -N linking them are part of an heterocycloalkyl selected from azetidinyl, pyrrolidinyl, imidazolinyl, pyrolidin-2-one, morpholinyl, thiomorpholinyl, piperidinyl, piperidin-2-one, piperazinyl, N-alkyl-piperazinyl;
**R8** and **R9** are independently from each other hydrogen, halogen, alkyl, CN, CF₃, OCF₃, heterocycloalkyl, N(alkyl)₂ or alkoxy;
as well as pharmaceutically acceptable salt thereof, a prodrug of the compound or the salt, or a solvate or hydrate of the compound, the salt or the prodrug.

2. A compound according to claim 1, wherein **A** is **X-R1** with:
**R¹** is alkyl, aryl or heteroaryl;
**X** and **R3** to **R9** are as defined in claim 1;
as well as pharmaceutically acceptable salt thereof, a prodrug of the compound or the salt, or a solvate or hydrate of the compound, the salt or the prodrug.

3. A compound according to claim 1, wherein **A** is **N(R1) (R2)** with:
**R1** to **R9** are as defined in claim 1;
as well as pharmaceutically acceptable salt thereof, a prodrug of the compound or the salt, or a solvate or hydrate of the compound, the salt or the prodrug.

4. A compound according to claim 1 or 2, wherein **A** is **X-R1** with:
**X, R1,** and **R3** to **R7** groups are as defined in claim 1 or 2;
**R8** and **R9** are hydrogen.

5. A compound according to claim 1 or 3, wherein A is **N(R1)(R2)** with:
**R1** to **R7** groups are as defined in claim 1 or 3;
**R8** and **R9** are hydrogen.

6. A compound according to claim 1 or 2, wherein **A** is **X-R1** with:
**A** is as defined in claims 1 and 2;
**R3** to **R9** are hydrogen.

7. A compound according to claim 1 or 3, wherein **A** is **N(R1)(R2)** with:
**A** is as defined in claims 1 and 3;
**R3** to **R9** are hydrogen.

8. Compound according to anyone of claims 1 to 7 selected from the group consisting of:
*2-[1-(2-Morpholin-4-yl-ethoxy)-naphthalen-2-ylamino]-acetamide*
*2-(1-Morpholin-4-yl-naphthalen-2-ylamino)-acetamide*

9. Use of a compound according to anyone of claims 1 to 8 in the manufacture of a drug for use in the treatment and/or prophylaxis of conditions and diseases linked to abnormalities in glutamatergic transmission.

10. A pharmaceutical composition comprising a pharmaceutically effective amount of a compound of formula (I) according to claim 1, or pharmaceutically acceptable salts thereof, in combination with a pharmaceutically acceptable carrier, diluent or excipient.
